# EUROPEAN PATENT SPECIFICATION

(11) **EP 4 231 935 B1**
(45) Date of publication and mention of the grant of the patent: **03.12.2025**
(21) Application number: 20957911.9
(22) Date of filing: 20.10.2020
(51) Int. Cl.: A61B 17/128, A61B 17/10, A61B 17/068, A61B 17/00

(54) **SURGICAL ACTUATOR WITH INTERCHANGEABLE APPLIER**
CHIRURGISCHER AKTUATOR MIT AUSTAUSCHBAREM APPLIKATOR
ACTIONNEUR CHIRURGICAL AVEC APPLICATEUR INTERCHANGEABLE

(43) Date of publication of application: 30.08.2023
(73) Proprietor: Taiwan Surgical Corporation, Hsinchu County 302 (TW)
(72) Inventor: SUN, Chien-Wei, Hsinchu City 300, Taiwan (CN); LIN, Wu-Shen, Taoyuan City 326, Taiwan (CN)
(74) Representative: Cabinet Chaillot
(86) International application number: PCT/AU2020/051131
(87) International publication number: WO 2022/082250

(56) References cited:
- EP-B1- 2 635 220
- WO-A2-2019/089426
- US-A- 5 749 881
- US-A1- 2008 021 278
- US-A1- 2008 021 278
- US-A1- 2016 174 969
- US-A1- 2020 261 095

## Description

### FIELD OF INVENTION

The present disclosure generally relates to an actuator that is adapted to a surgical device with interchangeable mechanism, and more particularly it relates to a surgical actuator with interchangeable applier.

The present disclosure has been developed primarily to provide an actuator for surgical device, and will be described hereinafter with reference to this application.

### BACKGROUND OF THE INVENTION

Conventional surgical actuator, such as surgical clip, is provided with a body including a handle and a trigger with extending a long tube from the body. A front end of the long tube is pivoted with a jaw that can be driven by the trigger in an open or closed status. When using the said surgical actuator, user could hold the handle and pulls the trigger for actuating the plastic staple on patient's tissue.

Although the said surgical actuator can be easily used to apply plastic staples on tissues, having the structure that is a non-detachable design for which it can only be used to apply plastic staples for single purpose. When the user needs to use other functions from the said surgical actuator, they have no choice to use anther surgical device or apparatus which is considered high cost and will cause a lot of time and troublesome for changing the device. Hence, the present disclosure seeks to provide a surgical actuator with interchangeable applier that will overcome or substantially ameliorate at least one or more of the deficiencies of a prior art, or to at least provide an alternative solution to the problems. It is to be understood that, if any prior art information is referred to herein, such reference does not constitute an admission that the information forms part of the common general knowledge in the art.

In addition, each of US 2008/021278. EP 2 635 220, and US 5 749 881 also disclose a conventional surgical instrument.

### SUMMARY OF THE INVENTION

The invention relates to a surgical actuator with an interchangeable applier and is defined by the features of independent claim 1. Preferred embodiments are given in the dependent claims. In order to solve the problems bring by the conventional surgical actuator with non-detachable applier as described above, the present disclosure is presented.

According to a first aspect of the present disclosure, the surgical actuator with interchangeable applier comprises: an actuator body and an interchangeable applier. An actuator body comprises an outer tube extended in a back and forth direction. An installation section is formed at a front end of the outer tube. A button is provided on the installation section with its bottom extruded inwardly from an inner surface of the installation section. A positioning groove is formed at the inner wall of the installation section. A driving rod is provided to slide inside the outer tube. A neck portion is formed at a front end of the driving rod. An enlarged pivot portion is formed at a front end of the neck portion. The enlarged pivot portion is extended and reaches the installation section. A long tube is mounted with the interchangeable applier. A front end of the long tube is pivotally connected with an applier. A rear end of the long tube forms an insertion section which is inserted into the installation section at a front end of the actuator body. A long groove is formed on an outer surface of the insertion section. A recess is formed at a front end of the long groove. The button is received by the recess. A buckle is formed on the outer surface of the insertion section. The buckle is embedded into the positioning groove. A connecting rod is placed and is slidable inside the long tube. A front end of the connecting rod is connected with the applier and the applier is driven by the connecting rod. A round recess is formed at a rear end of the connecting rod for receiving the enlarged pivot portion. A slit is formed at the rear end of the connecting rod. The slit is penetrated through the connecting rod and connects a rear side of the round recess. The slit is enlarged at its middle portion of to form a round insert hole. A diameter of the round insert hole is smaller than a diameter of the round recess. A guiding sphere surface is formed inwardly at the rear end of the connecting rod. The enlarged pivot portion is configured to be received by the round recess by pushing aside opposite sides of the round insert hole along the guiding sphere surface.

When using the present disclosure, a user, such as a surgeon, could hold the actuator body and insert the interchangeable applier into an opening on a patient reaching an area needs to be treated. The user holds the handle and presses the trigger. The trigger will drive the driving rod moving forward. As the driving rod moving forward, the enlarged pivot portion pushes the connecting rod forward and drives the applier in a close or open status. As the user loosen the trigger, the block will be moved backward to make the trigger and the driving rod back to original place. In the middle of the driving rod moving to its original place, the enlarged pivot portion will embedded into the round recess and pulling back the connecting rod. At the same time, the connecting rod will also be driven to move back to its original place and status and the applier will remain open.

When the user needs to change the interchangeable applier of the present disclosure, the user may rotate the interchangeable applier for the buckle of the interchangeable applier being detached from the positioning groove and make the button moving from the recess to the front end of the long groove. The two cutting planes and two opposite side of the slit will be correspondingly positioned. The user may further pull the interchangeable applier forward, the buckle will move backwardly along the long groove. The enlarged pivot portion will also leave the round recess by pushing the round insert hole and moving backward. By doing above steps, the interchangeable applier could be hence detached from the actuator body and achieve the function of interchangeable mechanism. The present disclosure is able to install different types of appliers as desire with different functions. A single actuator body could adapt with many different kinds of appliers providing various functions.

Many of the attendant features and advantages of the present disclosure will become better understood with reference to the following detailed description considered in connection with the accompanying figures and drawings.

### BRIEF DESCRIPTION OF THE DRAWINGS

The steps and the technical means adopted by the present disclosure to achieve one or more of the above objectives can be best understood by referring to the following detailed description of the preferred embodiments and the accompanying drawings.
Fig. 1 is perspective view of a preferred embodiment in accordance with the present.
Fig. 2 is a illustration of the interchangeable applier in a preferred embodiment in accordance with the present.
Fig. 2A is an enlarged illustration of Fig. 2.
Fig. 3 is a exploded view of the interchangeable applier in a preferred embodiment in accordance with the present.
Fig. 3A is an enlarged illustration of Fig. 2.
Fig. 4 is a cross-section view of a preferred embodiment in accordance with the present.
Fig. 4A is a enlarged illustration of Fig. 4.
Fig. 5 is a cross-section view of the button in a preferred embodiment in accordance with the present.
Fig. 6 is a enlarged pivot portion in a preferred embodiment in accordance with the present.
Fig. 7A and Fig. 7B are illustrations for installing the button in a preferred embodiment in accordance with the present.
Fig. 8A and Fig. 8B illustrations for installing the enlarged pivot portion in a preferred embodiment in accordance with the present.

### DETAILED DESCRIPTION OF THE PREFERRED EMBODIMENTS

Reference will now be made in detail to the preferred embodiments of the present disclosure, examples of which are illustrated in the accompanying drawings. Wherever possible, the same reference numbers are used in the drawings and the description to refer to the same or like parts. It is not intended to limit the method by the exemplary embodiments described herein. In the following detailed description, for the purposes of explanation, numerous specific details are set forth in order to provide a thorough understanding of the disclosed embodiments. It will be apparent, however, that one or more embodiments may be practiced without these specific details. As used in the description herein and throughout the claims that follow, the meaning of "a", "an", and "the" may include reference to the plural unless the context clearly dictates otherwise. Also, as used in the description herein and throughout the claims that follow, the terms "comprise or comprising", "include or including", "have or having", "contain or containing" and the like are to be understood to be open-ended, i.e., to mean including but not limited to. As used in the description herein and throughout the claims that follow, the terms "layer" may infer to be a film or a membrane.

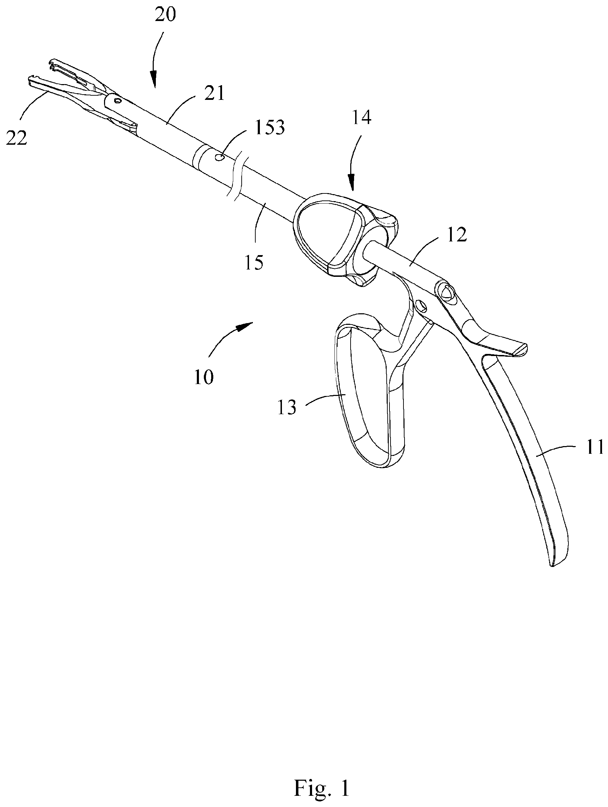

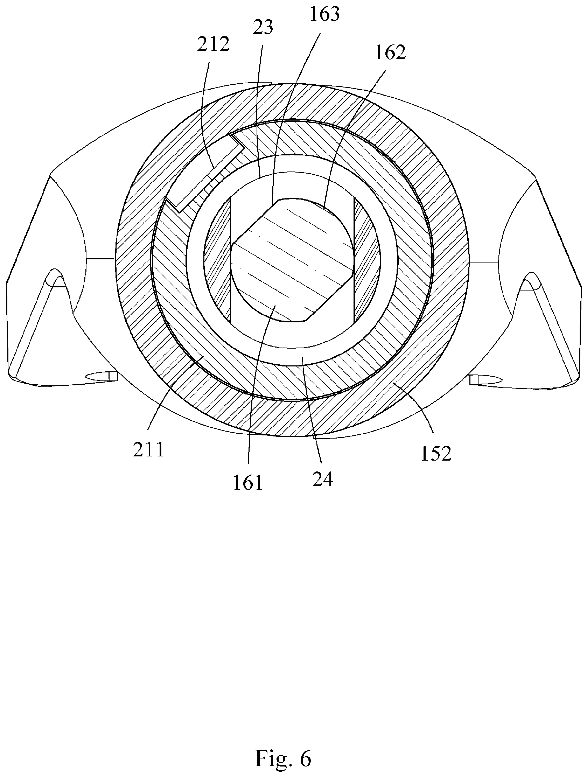

With reference to Figs. 1 to 6, one preferred embodiment of the present invention provides a surgical actuator with an interchangeable applier 20 comprising an actuator body 10 and an interchangeable applier 20 mounted at a front end of the actuator body 10.

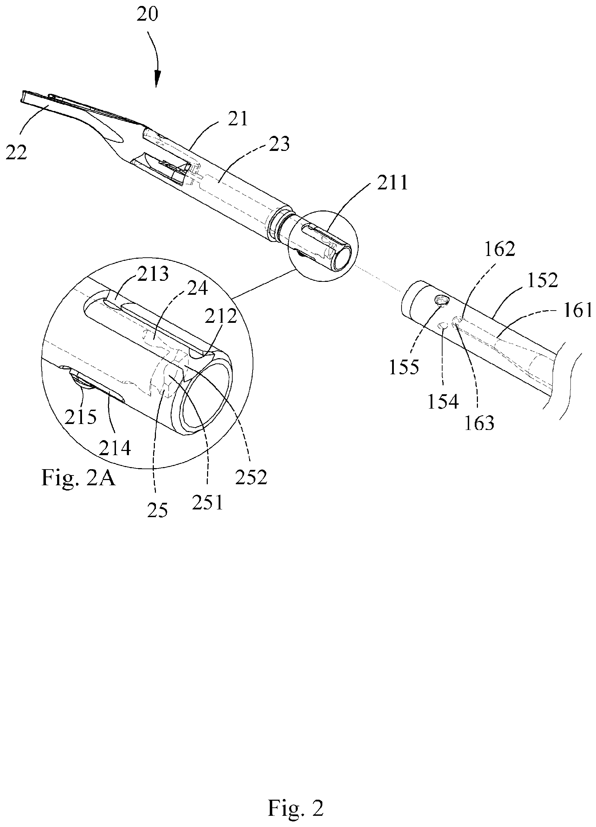

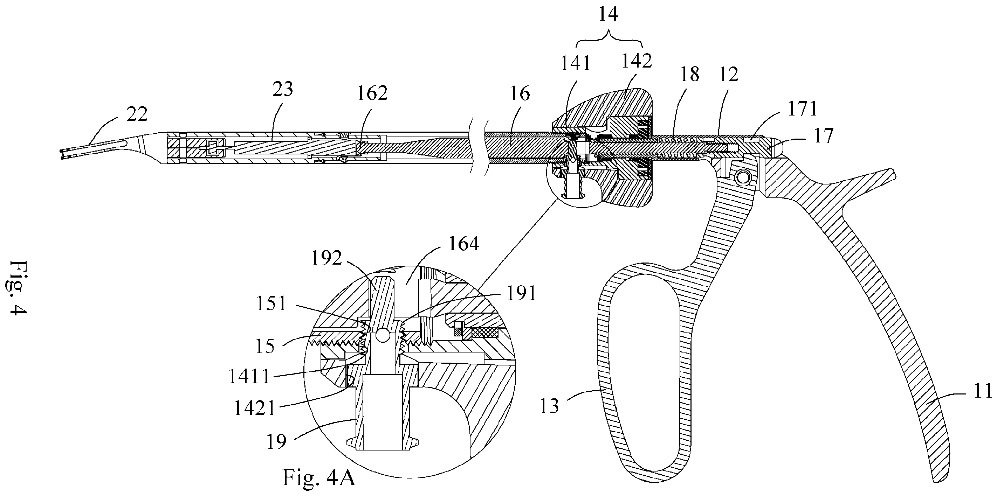

With reference to Figs. 2 to 4, the actuator body 10 comprises a handle 11 extended forwardly with a main body 12. A trigger 13 projected in O-shape in this embodiment is pivotally connected with a bottom side of the main body 12. A rotatable element 14 is mounted on a front end of the main body 12. The rotatable element 14 comprises a rotary sleeve 141 inside a rotary body 142. The rotary sleeve 141 is ratably mounted at the front end of the main body 12. With reference to Fig. 4A, a first perforation 1411 is formed at a front end of the rotary sleeve 141 in radial direction. The rotary body 142 is sleeved by the rotary sleeve 141 and a second perforation 1421 is formed on the rotary body 142 in radial direction.

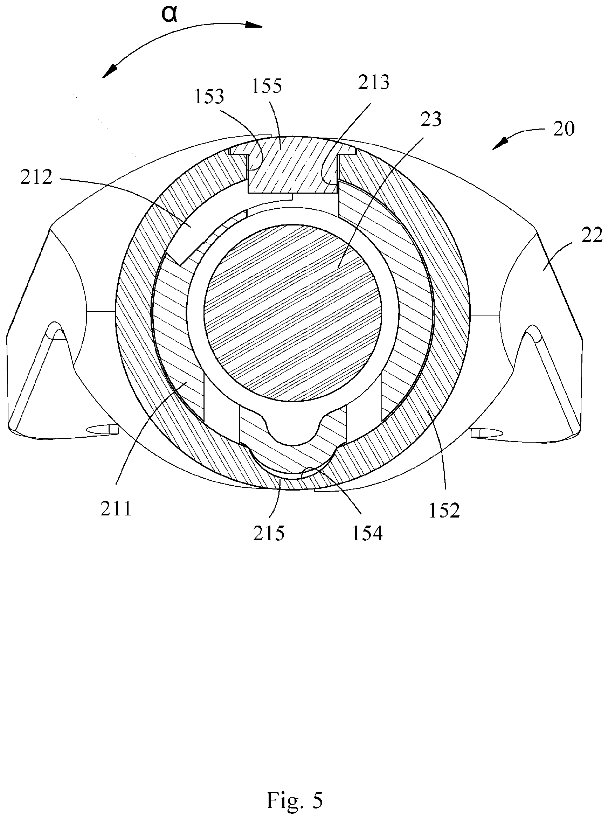

An outer tube 15 is screwed at the front end of the rotary sleeve 141. The outer tube 15 is a long straight tube extending from the front end of the rotary sleeve 141. At the first perforation 1411, a screw hole 151 is perforated in radial direction at a rear end of the outer tube 15. An installation section 152 is formed at the front end of the outer tube 15. A hole 153 is formed at a surface of the installation section 152. A button 155 is embedded in the hole 153 with its bottom extruded inwardly from an inner surface of the installation section 152 as shown in Fig. 5. A positioning groove 154 is formed at the inner wall of the installation section 152 opposite from the surface where the hole 153 is.

In Fig. 4, a driving rod 16 is able to slide inside the outer tube 15 in a front and rear direction. A rear end of the driving rod 16 is installed or sleeved within the main body 12. A neck portion 161 with decreasing diameter is formed at a front end of the driving rod 16. An enlarged pivot portion 162 is formed at a front end of the neck portion 161. A diameter of the enlarged pivot portion 162 is greater than a diameter of the neck portion 161. The enlarged pivot portion 162 is extended and reached to the installation section 152. Two cutting planes 163 are parallelly formed on an opposite sides of the enlarged pivot portion 162. Corresponding to a position of the screw hole 151, a positioning bore 164 is penetrated in a radial direction through the driving rod 16. The positioning bore 164 is a long bore extended in a back and forth direction.

With reference to Fig. 4, a block 17 is screwed with the rear end of the driving rod 16. A ring groove 171 is formed surrounded the block 17. A tip of the trigger 13 is extended into the ring groove 17. Thus, when the driving rod 16 rotates at any angle along its axis, the trigger 13 is able to move the block 17 and activate the driving rod 16 for pushing it forward. A spring 18 is mounted at a front end of the block 17 inside the main body 12 for recovering or rebounding the driving rod 16, the block 17 and the trigger 13 back to their original position or status.

With reference to Fig. 4A, a tubular fixing element 19 is partially placed inside the second perforation 1421 with a screw tube 191. The screw tube 191 is extended from the first perforation 1411 and is screwed with the screw hole 151 of the outer tube 15. A positioning pin 192 is extended from the screw tube 191 and reached to the positioning bore 164. The positioning pin 192 is extended into a front side of the positioning bore 164 of the driving rod 16 making a rotating relation and a position between the driving rod 16 and the outer tube 15 to be fixed. When the driving rod 16 is driven by the trigger 13, the driving rod 16 can be moved back and forth in a range which is limited by the positioning bore 164 can be moved along with the positioning pin 192.

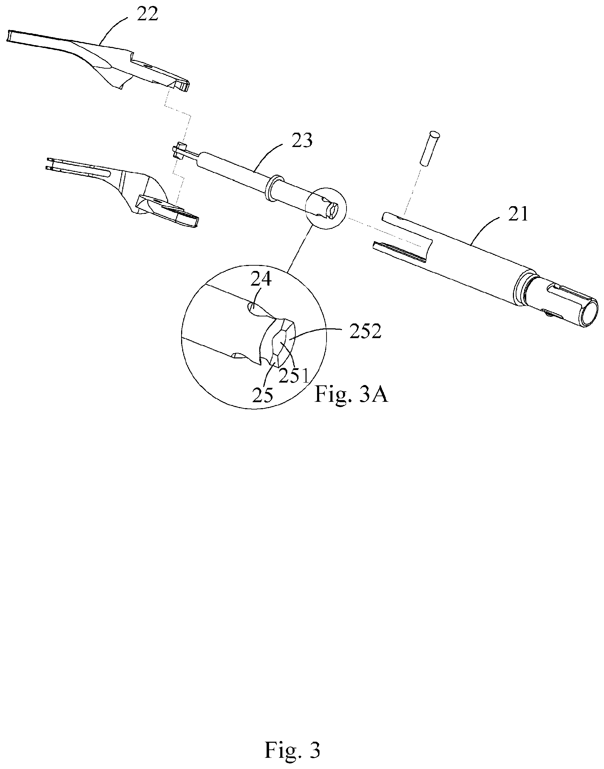

A connecting rod 23 is placed and can be slide back and forth inside the long tube 21. A front end of the connecting rod 23 is connected with the applier 22 so as to be driven by the connecting rod 23 in a close or open status when the connecting rod 23 slide back and forth. A round recess 24 is formed at a rear end of the connecting rod 23. A diameter of the round recess 24 shares the same diameter of the enlarged pivot portion 162 which the round recess 24 could receive the enlarged pivot portion 162. A slit 25 is formed at the rear end of the connecting rod 23. The slit 25 is penetrated through the connecting rod 23 and connects a rear side of the round recess 24. An opposite surface of the slit 25 is parallel to each other and a distance between the opposite surfaces of the slit 25 is smaller than the diameter of the round recess 24. Also the distance between the opposite surfaces of the slit 25 is smaller than the distance of two cutting planes 163 of the enlarged pivot portion 162. At middle of the slit 25 is enlarged to form a round insert hole 251 as shown in Fig. 3. A diameter of the round insert hole 251 is also smaller than a diameter of the round recess 24. A guiding sphere surface 252 is formed inwardly at the rear end of the connecting rod 23.

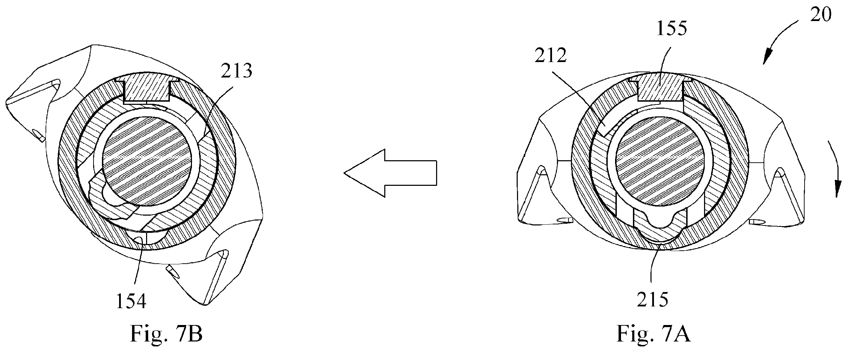

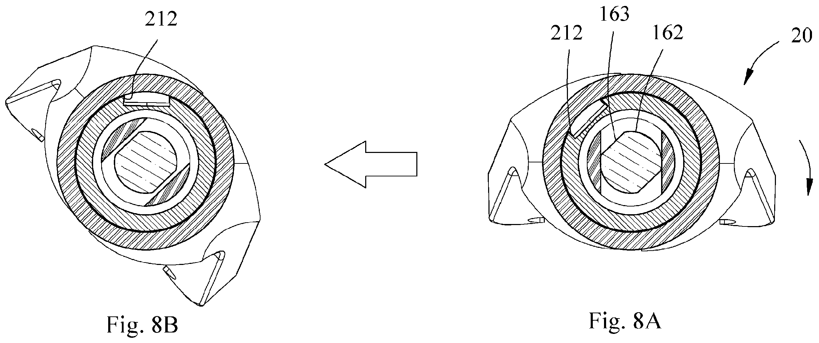

Please referring to Figs. 5 and 7 to 8, when the interchangeable applier 20 is rotated, the button 155 is moved from the recess 213 to the front end of the long groove 212. The button 155 is rotated in a rotary angle α within the recess 213. When the interchangeable applier 20 is installed at the front end of the actuator body 10, two cutting planes 163 and two opposite sides of the slit 25 are also rotated in the rotary angle α. Thus, as the interchangeable applier 20 rotating and the button 155 moving from the recess 213 to the front end of the long groove 212, two cutting planes 163 and two opposite sides of the slit 25 will be correspondingly positioned. As such, no matter when the enlarged pivot portion 162 is moved out of or into the round recess 24, the interchangeable applier 20 could be installed more easily as a contact area and a applied force can be reduced when the enlarged pivot portion 162 passes through the round insert hole 251.

With reference to Figs. 3 and 4, when using the preferred embodiment of the present invention, a user holds the handle 11 and presses the trigger 13. The trigger 13 will drive the driving rod 16 moving forward. As the driving rod 16 moving forward, the enlarged pivot portion 162 pushes the connecting rod 23 forward and drives the applier 22 in a close or open status. As the user loosen the trigger 13, the spring 18 will push the block 17 moving backward to make the trigger 13 and the driving rod 16 back to original place. In the middle of the driving rod 16 moving to its original place, the enlarged pivot portion 162 will embedded into the round recess 24 and pulling back the connecting rod 23. At the same time, the connecting rod 23 will also be driven to move back to its original place and status and the applier 22 will remain open.

With reference to Figs. 2, 7A to 8B, when the user needs to change the interchangeable applier 20, the user may rotate the interchangeable applier 20 first for the buckle 215 of the interchangeable applier 20 detached from the positioning groove 154 and make the button 155 moving from the recess 231 to the front end of the long groove 212. The two cutting planes 163 and two opposite side of the slit 25 will be correspondingly positioned. The user may further pulling the interchangeable applier 20 forward, the buckle 215 will move backwardly along the long groove 212. The enlarged pivot portion 162 will also leave the round recess 24 by pushing the round insert hole 251 and moving backward. By doing above steps, the interchangeable applier 20 could be hence detached from the actuator body 10. The present disclosure is able to install different types of appliers 20 as desire with different functions. A single actuator body 10 could adapt with many different kinds of appliers 20 with various functions.

The above specification, examples, and data provide a complete description of the present disclosure and use of exemplary embodiments. Although various embodiments of the present disclosure have been described above with a certain degree of particularity, or with reference to one or more individual embodiments, those with ordinary skill in the art could make numerous alterations or modifications to the disclosed embodiments without departing from or scope of this disclosure.

## Claims

1. A surgical actuator with an interchangeable applier (20) comprising an actuator body (10) and an interchangeable applier (20), wherein:
the actuator body (10) comprises an outer tube (15) extended in a back and forth direction;
an installation section (152) is formed at a front end of the outer tube (15);
a button (155) is provided on the installation section (152) with its bottom extruded inwardly from an inner surface of the installation section (152);
a positioning groove (154) is formed at the inner wall of the installation section (152);
a driving rod (16) is configured to slide inside the outer tube (15);
a neck portion (161) is formed at a front end of the driving rod (16);
an enlarged pivot portion (162) is formed at a front end of the neck portion (161);
the enlarged pivot portion (162) is extended and reaches the installation section (152);
a long tube (21) is mounted with the interchangeable applier (20);
a front end of the long tube (21) is configured to be pivotally connected with an applier (22);
a rear end of the long tube (21) forms an insertion section which is inserted into the installation section (152) at a front end of the actuator body (10);
a long groove (212) is formed on an outer surface of the insertion section;
a recess (213) is formed at a front end of the long groove (212);
the button (155) is received by the recess (213);
a buckle (215) is formed on the outer surface of the insertion section;
the buckle (215) is embedded into the positioning groove (154);
a connecting rod (23) is placed and is configured to be slidable inside the long tube (21);
a front end of the connecting rod (23) is connected with the applier (22) and the applier (22) is configured to be driven by the connecting rod (23)
a round recess (24) is formed at a rear end of the connecting rod (23) for receiving the enlarged pivot portion (162);
a slit (25) is formed at the rear end of the connecting rod (23);
the slit (25) is penetrated through the connecting rod (23) and connects a rear side of the round recess (24); **characterized in that**
the slit (25) is enlarged at its middle portion to form a round insert hole (251);
a diameter of the round insert hole is smaller than a diameter of the round recess (24);
a guiding sphere surface (252) is formed inwardly at the rear end of the connecting rod (23); and
the enlarged pivot portion is configured to be received by the round recess (24) by pushing aside opposite sides of the round insert hole along the guiding sphere surface (252).

2. The actuator as claimed in claim 1, wherein:
the button (155) is configured to be rotated in a rotary angle (α) from the recess (24) to the front end of the long groove (121);
two cutting planes (163) are parallelly formed on an opposite sides of the enlarged pivot portion (162);
the distance of two cutting planes (163) of the enlarged pivot portion (162) is larger than a distance between the opposite surfaces of the slit (25); and
two cutting planes (163) and two opposite sides of the slit (25) are configured to be rotated in the rotary angle (α).

3. The actuator as claimed in claim 1, wherein:
the actuator body (10) comprises a handle (11) extended forwardly with a main body (12);
a trigger (13) is pivotally connected with a bottom side of the main body (12);
a rotatable element (14) is mounted on a front end of the main body (12);
the outer tube (15) is connected from the rotatable element (14): and
the driving rod (16) is configured to be driven by the trigger (13).

4. The actuator as claimed in claim 2, wherein:
the actuator body (10) comprises a handle (11) extended forwardly with a main body (12);
a trigger (13) is pivotally connected with a bottom side of the main body (12);
a rotatable element (14) is mounted on a front end of the main body (12);
the outer tube (15) is connected from the rotatable element (14): and
the driving rod (16) is driven by the trigger (13).

5. The actuator as claimed in claim 3, wherein:
the rotatable element (14) comprises a rotary sleeve (141) inside a rotary body (142);
the rotary sleeve (141) is ratably mounted at the front end of the main body (12);
the outer tube (15) is screwed at the front end of the rotary sleeve (141);
a screw hole (151) is perforated in radial direction at a rear end of the outer tube (15);
a first perforation (1411) is formed at a front end of the rotary sleeve (141);
the rotary body (142) is sleeved by the rotary sleeve (141);
a second perforation (1421) is formed on the rotary body (142);
a tubular fixing element (19) is partially placed inside the second perforation (1421) with a screw tube (191);
the screw tube (191) is extended from the first perforation and is screwed with the screw hole of the outer tube (15);
a positioning bore (164) is penetrated in a radial direction through the driving rod (16);
the positioning bore (164) is a long bore extended in a back and forth direction; and
a positioning pin (192) is extended from the screw tube (191) and reached to the positioning bore (164).

6. The actuator as claimed in claim 4, wherein:
the rotatable element (14) comprises a rotary sleeve (141) inside a rotary body (142);
the rotary sleeve (141) is ratably mounted at the front end of the main body (12);
the outer tube (15) is screwed at the front end of the rotary sleeve (141);
a screw hole (151) is perforated in radial direction at a rear end of the outer tube (15);
a first perforation (1411) is formed at a front end of the rotary sleeve (141);
the rotary body (142) is sleeved by the rotary sleeve (141);
a second perforation (1421) is formed on the rotary body (142);
a tubular fixing element (19) is partially placed inside the second perforation (1421) with a screw tube (191);
the screw tube (191) is extended from the first perforation and is screwed with the screw hole of the outer tube (15);
a positioning bore (164) is penetrated in a radial direction through the driving rod (16);
the positioning bore (164) is a long bore extended in a back and forth direction; and
a positioning pin (192) is extended from the screw tube (191) and reached to the positioning bore (164).

## Patentansprüche

1. - Chirurgischer Aktuator mit einem austauschbaren Applikator (20), umfassend einen Aktuatorkörper (10) und einen austauschbaren Applikator (20), wobei:
der Aktuatorkörper (10) ein äußeres Rohr (15) umfasst, das sich in einer Hin- und Herrichtung erstreckt;
an einem vorderen Ende des äußeren Rohrs (15) ein Installationsbereich (152) gebildet ist;
ein Knopf (155) an dem Installationsbereich (152) bereitgestellt ist, dessen Boden von einer Innenfläche des Installationsbereichs (152) einwärts hervorsteht;
an der Innenwand des Installationsbereichs (152) eine Stellnut (154) gebildet ist;
eine Antriebsstange (16) konfiguriert ist, um im Inneren des äußeren Rohrs (15) zu gleiten;
an einem vorderen Ende der Antriebsstange (16) ein Halsabschnitt (161) gebildet ist;
an einem vorderen Ende des Halsabschnitts (161) ein vergrößerter Zapfenabschnitt (162) gebildet ist;
der vergrößerte Zapfenabschnitt (162) ausgefahren wird und den Installationsbereich (152) erreicht;
ein langes Rohr (21) mit dem auswechselbaren Gerät (20) montiert wird;
ein vorderes Ende des langen Rohrs (21) konfiguriert ist, um schwenkbar mit einem Applikator (22) verbunden zu sein;
ein hinteres Ende des langen Rohrs (21) einen Einführungsabschnitt bildet, der an einem vorderen Ende des Aktuatorkörpers (10) in den Installationsbereich (152) eingeführt wird;
eine lange Nut (212) an einer Außenfläche des Einführungsabschnitts gebildet ist;
an einem vorderen Ende der langen Nut (212) eine Aussparung (213) gebildet ist;
der Knopf (155) von der Aussparung (213) aufgenommen wird;
eine Schnalle (215) an der Außenfläche des Einführungsabschnitts gebildet ist;
die Schnalle (215) in die Stellnut (154) eingelassen ist;
eine Verbindungsstange (23) platziert und konfiguriert ist, um im Inneren des langen Rohrs (21) gleitbar zu sein;
ein vorderes Ende der Verbindungsstange (23) mit dem Applikator (22) verbunden ist und der Applikator (22) konfiguriert ist, um von der Verbindungsstange (23) angetrieben zu werden;
an einem hinteren Ende der Verbindungsstange (23) eine runde Aussparung (24) zum Aufnehmen des vergrößerten Zapfenabschnitts (162) gebildet ist;
an dem hinteren Ende der Verbindungsstange (23) ein Schlitz (25) gebildet ist;
der Schlitz (25) die Verbindungsstange (23) durchdringt und eine Rückseite der runden Aussparung (24) verbindet ; **dadurch gekennzeichnet, dass**
der Schlitz (25) in seinem mittleren Abschnitt vergrößert ist, um ein rundes Einführloch (251) zu bilden;
der Durchmesser des runden Einführlochs kleiner ist als der Durchmesser der runden Aussparung (24);
eine Führungskugelfläche (252) einwärts an dem hinteren Ende der Verbindungsstange (23) gebildet ist; und
der vergrößerte Zapfenabschnitt konfiguriert ist, um von der runden Aussparung (24) aufgenommen zu werden, indem gegenüberliegende Seiten des runden Einführlochs entlang der Führungskugelfläche (252) zur Seite geschoben werden.

2. - Aktuator nach Anspruch 1, wobei:
der Knopf (155) konfiguriert ist, um in einem Drehwinkel (α) von der Aussparung (24) zu dem vorderen Ende der langen Nut (121) gedreht zu werden;
zwei Schnittebenen (163) parallel auf gegenüberliegenden Seiten des vergrößerten Zapfenabschnitts (162) gebildet sind;
der Abstand von zwei Schnittebenen (163) des vergrößerten Zapfenabschnitts (162) größer ist als ein Abstand zwischen den gegenüberliegenden Flächen des Schlitzes (25); und
zwei Schnittebenen (163) und zwei gegenüberliegende Seiten des Schlitzes (25) konfiguriert sind, um um den Drehwinkel (α) gedreht zu werden.

3. - Aktuator nach Anspruch 1, wobei:
der Aktuatorkörper (10) einen Griff (11) umfasst, der sich mit einem Hauptkörper (12) nach vorne erstreckt;
ein Auslöser (13) schwenkbar mit einer Unterseite des Hauptkörpers (12) verbunden ist;
ein drehbares Element (14) ist an einem vorderen Ende des Hauptkörpers (12) montiert ist;
das äußere Rohr (15) mit dem drehbaren Element (14) verbunden ist; und
die Antriebsstange (16) konfiguriert ist, um durch den Auslöser (13) angetrieben zu werden.

4. - Aktuator nach Anspruch 2, wobei:
der Aktuatorkörper (10) einen Griff (11) umfasst, der sich mit einem Hauptkörper (12) nach vorne erstreckt;
ein Auslöser (13) schwenkbar mit einer Unterseite des Hauptkörpers (12) verbunden ist;
ein drehbares Element (14) ist an einem vorderen Ende des Hauptkörpers (12) montiert ist;
das äußere Rohr (15) mit dem drehbaren Element (14) verbunden ist; und
die Antriebsstange (16) durch den Auslöser (13) angetrieben wird.

5. - Aktuator nach Anspruch 3, wobei:
das drehbare Element (14) eine Drehhülse (141) innerhalb eines Drehkörpers (142) umfasst;
die Drehhülse (141) drehbar an dem vorderen Ende des Hauptkörpers (12) montiert ist;
das äußeren Rohr (15) an das vordere Ende der Drehhülse (141) geschraubt ist;
ein Schraubenloch (151) in radialer Richtung an einem hinteren Ende des äußeren Rohrs (15) gebohrt ist;
eine erste Bohrung (1411) an einem vorderen Ende der Drehhülse (141) gebildet ist;
der Drehkörper (142) auf die Drehhülse (141aufgesteckt ist;
eine zweite Bohrung (1421) an dem Drehkörper (142) gebildet ist;
ein rohrförmiges Befestigungselement (19) mit einem Schraubrohr (191) teilweise in die zweite Bohrung (1421) eingesetzt ist;
das Schraubrohr (191) von der ersten Bohrung ausgeht und mit dem Schraubloch des äußeren Rohrs (15) verschraubt ist;
eine Stellbohrung (164) in radialer Richtung durch die Antriebsstange (16) gebohrt ist;
die Stellbohrung (164) eine lange Bohrung ist, die sich in einer Hin- und Herrichtung erstreckt; und
sich ein Stellstift (192) aus dem Schraubenrohr (191) erstreckt und die Stellbohrung (164) erreicht.

6. - Aktuator nach Anspruch 4, wobei:
das drehbare Element (14) eine Drehhülse (141) innerhalb eines Drehkörpers (142) umfasst;
die Drehhülse (141) drehbar an dem vorderen Ende des Hauptkörpers (12) montiert ist;
das äußeren Rohr (15) an das vordere Ende der Drehhülse (141) geschraubt ist;
ein Schraubenloch (151) in radialer Richtung an einem hinteren Ende des äußeren Rohrs (15) gebohrt ist;
eine erste Bohrung (1411) an einem vorderen Ende der Drehhülse (141) gebildet ist;
der Drehkörper (142) auf die Drehhülse (141) aufgesteckt ist;
eine zweite Bohrung (1421) an dem Drehkörper (142) gebildet ist;
ein rohrförmiges Befestigungselement (19) mit einem Schraubrohr (191) teilweise in die zweite Bohrung (1421) eingesetzt ist;
das Schraubrohr (191) von der ersten Bohrung ausgeht und mit dem Schraubloch des äußeren Rohrs (15) verschraubt ist;
eine Stellbohrung (164) in radialer Richtung durch die Antriebsstange (16) gebohrt ist;
die Stellbohrung (164) eine lange Bohrung ist, die sich in einer Hin- und Herrichtung erstreckt; und
sich ein Stellstift (192) aus dem Schraubenrohr (191) erstreckt und die Stellbohrung (164) erreicht.

## Revendications

1. - Actionneur chirurgical avec un applicateur interchangeable (20) comprenant un corps d'actionneur (10) et un applicateur interchangeable (20), dans lequel :
le corps d'actionneur (10) comprend un tube externe (15) étendu dans une direction en avant et en arrière ;
une section d'installation (152) est formée à une extrémité avant du tube externe (15) ;
un bouton (155) est disposé sur la section d'installation (152) avec son fond extrudé vers l'intérieur à partir d'une surface interne de la section d'installation (152) ;
une rainure de positionnement (154) est formée sur la paroi interne de la section d'installation (152) ;
une tige d'entraînement (16) est configurée pour coulisser à l'intérieur du tube externe (15) ;
une partie de col (161) est formée à une extrémité avant de la tige d'entraînement (16) ;
une partie pivot élargie (162) est formée à une extrémité avant de la partie de col (161) ;
la partie pivot élargie (162) est étendue et atteint la section d'installation (152) ;
un tube long (21) est monté avec l'applicateur interchangeable (20) ;
une extrémité avant du tube long (21) est configurée pour être reliée de manière pivotante à un applicateur (22) ;
une extrémité arrière du tube long (21) forme une section d'introduction qui est introduite dans la section d'installation (152) à une extrémité avant du corps d'actionneur (10) ;
une rainure longue (212) est formée sur une surface externe de la section d'introduction ;
un renfoncement (213) est formé à une extrémité avant de la rainure longue (212) ;
le bouton (155) est reçu par le renfoncement (213) ;
une boucle (215) est formée sur la surface externe de la section d'introduction ;
la boucle (215) est incorporée dans la rainure de positionnement (154) ;
une bielle (23) est positionnée et est configurée pour être apte à coulisser à l'intérieur du tube long (21) ;
une extrémité avant de la bielle (23) est reliée à l'applicateur (22) et l'applicateur (22) est configuré pour être entraîné par la bielle (23) ;
un renfoncement arrondi (24) est formé à une extrémité arrière de la bielle (23) pour recevoir la partie pivot élargie (162) ;
une fente (25) est formée à l'extrémité arrière de la bielle (23) ;
la fente (25) traverse la bielle (23) et relie un côté arrière du renfoncement arrondi (24) ; **caractérisé par le fait que**
la fente (25) est élargie à sa partie centrale pour former un trou d'introduction arrondi (251) ;
le diamètre du trou d'introduction arrondi est plus petit que le diamètre du renfoncement arrondi (24) ;
une surface sphérique de guidage (252) est formée vers l'intérieur à l'extrémité arrière de la bielle (23) ; et
la partie pivot élargie est configurée pour être reçue par le renfoncement arrondi (24) par écartement de côtés opposés du trou d'introduction arrondi le long de la surface sphérique de guidage (252).

2. - Actionneur selon la revendication 1, dans lequel :
le bouton (155) est configuré pour être tourné d'un angle de rotation (α) à partir du renfoncement (24) jusqu'à l'extrémité avant de la rainure longue (121) ;
deux plans de coupe (163) sont formés parallèlement sur des côtés opposés de la partie pivot élargie (162) ;
la distance des deux plans de coupe (163) de la partie pivot élargie (162) est supérieure à la distance entre les surfaces opposées de la fente (25) ; et
les deux plans de coupe (163) et deux côtés opposés de la fente (25) sont configurés pour être tournés de l'angle de rotation (α).

3. - Actionneur selon la revendication 1, dans lequel :
le corps d'actionneur (10) comprend une poignée (11) étendue vers l'avant avec un corps principal (12) ;
une gâchette (13) est reliée de manière pivotante à un côté inférieur du corps principal (12) ;
un élément rotatif (14) est monté sur une extrémité avant du corps principal (12) ;
le tube externe (15) est relié à l'élément rotatif (14) ; et
la tige d'entraînement (16) est configurée pour être entraînée par la gâchette (13).

4. - Actionneur selon la revendication 2, dans lequel :
le corps d'actionneur (10) comprend une poignée (11) étendue vers l'avant avec un corps principal (12) ;
une gâchette (13) est reliée de manière pivotante à un côté inférieur du corps principal (12) ;
un élément rotatif (14) est monté sur une extrémité avant du corps principal (12) ;
le tube externe (15) est relié à l'élément rotatif (14) ; et
la tige d'entraînement (16) est entraînée par la gâchette (13).

5. - Actionneur selon la revendication 3, dans lequel :
l'élément rotatif (14) comprend un manchon rotatif (141) à l'intérieur d'un corps rotatif (142) ;
le manchon rotatif (141) est monté de manière rotative à l'extrémité avant du corps principal (12) ;
le tube externe (15) est vissé à l'extrémité avant du manchon rotatif (141) ;
un trou de vissage (151) est perforé dans une direction radiale à une extrémité arrière du tube externe (15) ;
une première perforation (1411) est formée à une extrémité avant du manchon rotatif (141) ;
le corps rotatif (142) est emmanché sur le manchon rotatif (141) ;
une seconde perforation (1421) est formée sur le corps rotatif (142) ;
un élément de fixation tubulaire (19) est partiellement positionné à l'intérieur de la seconde perforation (1421) avec un tube de vissage (191) ;
le tube de vissage (191) est étendu à partir de la première perforation et est vissé au trou de vissage du tube externe (15);
un alésage de positionnement (164) est percé dans une direction radiale à travers la tige d'entraînement (16) ;
l'alésage de positionnement (164) est un alésage long étendu dans une direction en avant et en arrière ; et
une goupille de positionnement (192) est étendue à partir du tube de vissage (191) et atteint l'alésage de positionnement (164).

6. - Actionneur selon la revendication 4, dans lequel :
l'élément rotatif (14) comprend un manchon rotatif (141) à l'intérieur d'un corps rotatif (142) ;
le manchon rotatif (141) est monté de manière rotative à l'extrémité avant du corps principal (12) ;
le tube externe (15) est vissé à l'extrémité avant du manchon rotatif (141) ;
un trou de vissage (151) est perforé dans une direction radiale à une extrémité arrière du tube externe (15) ;
une première perforation (1411) est formée à une extrémité avant du manchon rotatif (141) ;
le corps rotatif (142) est emmanché sur le manchon rotatif (141) ;
une seconde perforation (1421) est formée sur le corps rotatif (142) ;
un élément de fixation tubulaire (19) est partiellement positionné à l'intérieur de la seconde perforation (1421) avec un tube de vissage (191) ;
le tube de vissage (191) est étendu à partir de la première perforation et est vissé au trou de vissage du tube externe (15) ;
un alésage de positionnement (164) est percé dans une direction radiale à travers la tige d'entraînement (16) ;
l'alésage de positionnement (164) est un alésage long étendu dans une direction en avant et en arrière ; et
une goupille de positionnement (192) est étendue à partir du tube de vissage (191) et atteint l'alésage de positionnement (164).
